(19) Europäisches Patentamt
European Patent Office
Office européen des brevets

(11) Veröffentlichungsnummer: **0 027 953**
**B2**

# (12) NEUE EUROPÄISCHE PATENTSCHRIFT

(45) Veröffentlichungstag der neuen Patentschrift: **05.11.86**

(51) Int. Cl.[4]: **C 07 C 125/065,** C 07 C 125/073

(21) Anmeldenummer: **80106248.0**

(22) Anmeldetag: **15.10.80**

(54) **Verfahren zur Herstellung von N,O-disubstituierten Urethanen.**

(30) Priorität: **27.10.79 DE 2943551**

(43) Veröffentlichungstag der Anmeldung:
**06.05.81 Patentblatt 81/18**

(45) Bekanntmachung des Hinweises auf die Patenterteilung:
**16.02.83 Patentblatt 83/7**

(45) Bekanntmachung des Hinweises auf die Entscheidung über den Einspruch:
**05.11.86 Patentblatt 86/45**

(84) Benannte Vertragsstaaten:
**AT BE CH DE FR GB IT LI NL SE**

(56) Entgegenhaltungen:
**EP-A-0 018 581**
**EP-A-0 018 583**
**EP-B-0 018 586**
**EP-B-0 018 588**
**US-A-2 409 712**
**US-A-2 677 698**
**US-A-2 806 051**

**Methoden der organischen Chemie (Houben-Weyl) Band VIII, Georg Thieme Verlag, Stuttgart, 1952, Seite 140 und 161**
**Journal of the American Chemical Society, Band 76 (15), Seiten 3977 bis 3978, 1954**

(73) Patentinhaber: **BAYER AG, Konzernverwaltung RP Patentabteilung, D-5090 Leverkusen 1 Bayerwerk (DE)**

(72) Erfinder: **Heitkämper, Peter, Dr., Fliederweg 14, D-4047 Dormagen 11 (DE)**
Erfinder: **König, Klaus, Dr., Heymannstrasse 10, D-5090 Leverkusen 1 (DE)**
Erfinder: **Findeisen, Kurt, Dr., In der Follmuehle 10, D-5068 Odenthal 2 (DE)**
Erfinder: **Fauss, Rudolf, Dr., Gerstenkamp 10, D-5000 Köln 80 (DE)**
Erfinder: **Sundermann, Rudolf, Dr., Treptower Strasse 5, D-5090 Leverkusen 1 (DE)**

EP 0 027 953 B2

**Beschreibung**

Verfahren zur Herstellung von N,O-disubstituierten Urethanen

Die vorliegende Erfindung betrifft ein verbessertes Verfahren zur Herstellung von Urethanen durch Umsetzung von Harnstoff oder Polyureten mit Aminen und Alkoholen unter Verwendung bestimmter, nachstehend näher beschriebener zusätzlicher Reaktionspartner für diese Umsetzung.

Urethane entstehen bekanntlich u. a. durch Umsetzung von organischen Isocyanaten mit Alkoholen. Diese Umsetzung ist reversibel, d. h. Urethane können thermisch in das ihnen zugrundeliegende Isocyanat und den ihnen zugrundeliegenden Alkohol gespalten werden (vgl. z. B. US-PS 24 09712). Thermisch in Isocyanate spaltbare Urethane sind daher interessante Ausgangsmaterialien zur Herstellung dieser Isocyanate, die bislang weltweit in größtem Umfang durch Umsetzung von primaren Aminen mit Phosgen hergestellt werden. Die phosgenfreie Herstellung von Urethanen und deren anschließende thermische Spaltung stellt eine interessante Alternative zu der genannten großtechnischen Isocyanatherstellung dar. Eine Methode zur phosgenfreien Herstellung von Urethanen besteht in der Umsetzung von Harnstoff mit Aminen und Alkohol (vgl. z. B. US-PS 2409712 oder JS-PS 28 05051). Diese Methoden des Standes der Technik sind jedoch mit dem Nachteil behaffet, daß die Urethane nur in unzureichender Ausbeute und in stark mit Nebenprodukten verunreinigter Form erhalten werden.

Es war die Aufgabe der vorliegenden Erfindung, das bekannte Vedahren zur Herstellung von Urethanen durch Umsetzung von Harnstoff mit Alkoholen und Aminen so zu verbessern, daß die N,Odisubstituierten Urethanne, bezogen auf eingesetzten Harnstoff. in verbesserter Ausbeute erhalten werden.

Überraschenderweise wurde nunmehr gefunden, daß diese Aufgabe durch Mitverwendung von weiteren, nachstehend näher beschriebenen, Carbonylgruppen aufweisenden Ausgangsmaterialien gelöst werden kann.

Gegenstand der vorliegenden Erfindung ist ein Verfahren zur Herstellung von Urethanen der allgemeinen Formel

$$R_1\left[NH-\overset{O}{\overset{\|}{C}}-O-R_2\right]_n$$

in welcher

$R_1$ für einen gegebenenfalls substituierten aliphatischen Kohlenwasserstoffrest mit 1-18 Kohlenstoffatomen, einen gegebenenfalls substituierten cycloaliphatischen Kohlenwasserstoff rest mit 3 bis 18 Kohlenstoffatomen, einen gegebenenfalls substituerten aromatischen Kohlenwasserstoff rest mit 6-15 Kohlenstoffatomen, einen gegebenenfalls substituierten araliphatischen Kohlenwasserstoffrest mit 7 bis 14 Kohlenstoffatomen, oder für einen gegebenenfalls substituierten 5- oder 6-gliedrigen heterocyclischen Rest steht, der zusätzlich noch mit einem Benzolring anelliert sein kann,

$R_2$ für einen gegebenenfalls substituierten Alkylrest mit 1 bis 20 Kohlenstoffatomen, einen gegebenenfalls substituierten Cycloalkylrest mit 3 bis 15 Kohlenstoffatomen und einen gegebenenfalls substituierten Aralkylrest mit 7 bis 14 Kohlenstoffatomen und

n eine ganze Zahl von 1 bis 3 bedeuten, wobei im Falle von $n = 2$ oder3 zwischen 2 mit dem Rest $R_1$ verknüpften Urethangruppen mindestens 2 Kohlenstoffatome angeordnet sind. durch Umsetzung von

a) Harnstoff oder Polyureten der Formel

$H_2N\text{-}(CO\text{-}NH)_m\text{-}H$

in welcher

m für eine ganze Zahl von 1 bis 4 steht, bzw. Gemische derartiger Verbindungen, mit

b) primären Aminen der Formel

$R_1(NH_2)_n$

in welcher

$R_1$ und n die bereits genannte Bedeutung haben, und

c) Alkoholen der Formel

$R_2\text{-}OH$

in welcher

$R_2$ die bereits genannte Bedeutung hat. im Temperaturbereich von 120 bis 350 °C, dadurch gekennzeichnet, daß man als weitere Reaktionspartner

d) N-unsubstituierte Urethane der Formel

in welcher

$R_3$ der Definition von $R_2$ entspricht, wobei $R_3$ und $R_2$ fur gleiche oder verschiedene Reste stehen, oder in welcher $R_3$ für einen gegebenenfalls Chlor oder $C_1$-$C_4$-Alkyl-substituierten, aromatischen Kohlenwasserstoff rest mit insgesamt 6 bis 15 Kohlenstoffatomen steht, und/oder

e) gegebenenfalls Urethan- oder primäre Aminoendgruppen aufweisende N-mono- oder N,N'disubstituierte Harnstoffe oder lineare Polyharnstoffe eines maximalen Molekulargewichts von 2 000, deren Harnstoff-, Urethan-bzw. Aminogruppen über Kohlenwasserstoffreste miteinander verknüpft sind. bzw. deren

Harnstoffgruppen mit Kohlenwasserstoffresten substituiert sind, die dem Rest $R_1$ entsprechen, und deren gegebenenfalls vorliegende endständige Urethangruppen am Sauerstoffatom mit Kohlenwasserstoffresten substituiert sind, die dem Rest $R_2$ entsprechen, mitverwendet, wobei die Herstellung von Arylmono-, -di- und/oder -polyurethanen durch Umsetzung von gegebenenfalls substituierten O-Alkyl-, O-Cycloalkyl- oder O-Aralkylcarbamidsäureestern mit primären aromatischen Mono-, Di- und/oder Polyaminen in Gegenwart von Alkoholen und Harnstoff ohne gleichzeitiqe Mitverwendung der unter e) genannten Ausgangsmaterialien ausgenommen ist, wobei die Reaktionspartner

a) bis e) zur Durchrührung des Verfahrens miteinander gemischt und auf die Reaktionstemperatur von 120°C bis 350°C erhitzt werden, und wobei die Amin-Komponente b), inklusive dem in der Komponente e) chemisch gebunden, der Amin-Komponente b) entsprechenden Amin, in einer 0,5-bis 4-fach stöchiometrischen Menge und die Alkohol-Komponente c) inklusive dem in den Komponente d) und e) chemisch gebunden Alkohol, falls dieser der Alkohol-Komponente c) entspricht, in einer 1-10-fach stöchiometrischen Menge, jeweils bezogen auf die in den Komponente a), d) und e) vorliegenden Caronylgruppen un die Komponente d) und e) in einer Menge von 0 bis 300 Gew.-%, bezogen auf die Menge der Komponente a) eingesets werden, mit der Einschränkung, daß die Gesamtmene der Komponenten d) und e) mindestens 10 Gew.-% beträgt.

Die veröffentlichten europäischen Patentanmeldungen 80 102 171.8 (0 018 581) und 80 102 179.1 (0 018 583) beschrieben berits die Herstellung von Aryl- mono- und/oder -polyurethanen durch Umsetzung von gegebenenfalls substituierten O-Alkyl-O-Cycloalkyl- oder O-Aralkylcarbidsäureestern mit primären aromatischen Mono- Di- und/oder Polyaminen in Gegenwart von Alkoholen und Harnstoff wobei gemäß der Patentanmeldung 80 102 179 1 auch Mitverwendung bestimmter Katalysatoren empfohlen wird. Diese Herstellung von Arylmono- und/oder -polyurethanen aus den genannten Ausgangsmaterialien in Abwesenheit der oben unter e) genannten Ausgangsmaterialien ist nicht Gegenstand der vorliegenden Erfindung,

Als Substituenten für d-ie aliphatischen oder cycloaliphatischen Reste $R_1$ bzw. $R_2$ kommen beispielsweise $C_6$-$C_{10}$-Aryloxy-, $C_1$-$C_6$-Alkoxy-, $C_1$-$C_6$-Alkoxy, $C_2$,$C_4$-alkoxy-, $C_1$-$C_6$-Acyl $C_1$,$C_6$-Alkylmercapto-, $C_6$-$C_{10}$-Arylmercapto-, $C_1$-$C_{12}$-Alkylcarbonyl-, Bis-($C_1$-$C_8$-Alkyl)-amino-, $C_1$-$C_6$-Acyl-amino-, Nitro-, Cyano- oder Rhodanoreste in Betracht.

Als Substituenten für die aromatischen oder araliphatischen Reste $R_1$ bzw. $R_2$kommen neben diesen Substituenten beispielsweise auch $C_1$-$C_{12}$-Alkyl-, $C_1$-$C_{12}$-Alkylsulfonsäureester-, oder Sulfonamidreste in Betracht.

Bevorzugte erfindu ngsgemäße Verfahrensprodukte sind jedoch solche der genannten allgemeinen Formel für welche

$R_1$ für einen aliphatischen Kohlenwasserstoffrest mit 3 bis 18 Kohlenstoffatomen, einen cycloaliphatischen Kohlenwasserstoffrest mit 6 bis 15 Kohlenstoffatomen oder einen gegebenenfalls Methyl-, Methoxy- oder Chlor-substituierten und gegebenenfalls Methylenbrücken aufweisenden aromatischen Kohlenwasserstoffrest mit 6 bis 15 Kohlenstoffatomen steht,

$R_2$ für einen $C_1$-$C_4$-Alkoxy- oder $C_1$-$C_4$-Alkoxy-$C_2$-$C_4$-alkoxysubstituierten oder unsubstituierten alophatischen Kohlenwasserstoffrest mit 1 bis 18, inbesondere 1 bis 4 Kohlenstoffatomen steht, wie er durch Entfernung der Hydroxylgruppe aus einem einwertigen unsubstituerten primären oder sekundären aliphatischen Alkol erhalten wird oder für einen Cyclohexyl- oder 2-Phenylethyl-Rest steht und

n 1 oder 2 bedutet.

Ausgangsmaterialien für das erfindungsgemäß Verfahren sind

a) Harnstoff, Bzw. Polyurete, insbesondere Biuret, Triuret oder Trauret oder Formel

$H_2N$-(CO-Nh)$_m$-H

in welcher

m für eine ganze Zahl von 1 bis 4 steht, bzw. Gemische derartiger Verbindungen Weitiere Ausgangsmaterialien für das erfindungsgemäß Verfahren sind

b) primäre Amine der Formel

$R_1(NH_2)_n$

in welcher

$R_1$ und n die bereits gennante Bedeutung haben.

Beispiele geeigneter Amine sind Methylamin, Ethylamin, Propylamin, Isopropylamin, Butylamin, iso-Butylamin, tert-Butylamin, Hexylamin, Dodecylamin, 2-Ethylhexylamin, Tetradecylamin, Hexadecylamin, Octadecylamin, Allylamin, 1,4-Diaminobutan, 1,6-Diaminohexan, 2,5-Dimethyl-2,5-hexandiamin, Trimethylhexamethylendiamin, 2-Methoxyetylamin, 3-Ethoxypropylamin, 3-Butoxypropylamin, 1,4-Butandiol-bis-(3-aminopropylether), 3-Aminopropansäure-2-methylpropylester, 6-Amino-hexanitril, Lysinester, 11-Aminoundecansäureester, Cyclohexylamin, Trimethylcyclohexylamin, 2-Norbornylmethylamin, Anilin, o,m,p-Chloranilin, 2,3-, 2,4-, 2,5-, 2,6-Dichloranilin, 3,4-Dichlornilin, p,o-Nitroanilin, m,o,p-Tolylamin, 3-Triflourmethylanilin, 3-Chlor-4-methylanilin, Benzylamin, Phenyl-cyclohexylamin, Naphthylamin, 1,4-Diaminocyclohexan, 2,4-, 2,6-Diamino-1-methylcoclohexan, 5-Amino-1-animomethyl-1,3,3-tri methylcyclohexan, 4,4'-Diaminodicyclohexylmethan, 4,4'-Diamino-3,3'-dimethyl-dicyclohexylmethan, 1,3-Diaminobenzol, 1,4-Diaminobenzon, 2-Chlor-1,4-diaminobenzol, 2,4-Diaminotoluol, 2,6-Diaminotoluol (und Gemische mit 2,4-), 2-(N-Ethyl-amino)-4-aminotoluol, 1,3-Bisaminomethylbenzol, 1,3-Bisaminomethyl-4,6-dimethylbenzol, 1,3-Diamino-2,6-(4,6)-diethyl-4-methylbenzol, 1,3-Diamino-2,4,6-triisopropylbenzol, 1,5-Diaminonaphthalin, 2,7-Diaminoaphtalin, Benzidin, 3,3'-Dichlorbenzidin, 4,4'-Diaminodiphenylmethan (und Rohwaren), 3,3'-Dichlor-4,4'-diamino-diphenylmethan, 2,2-Bis-(4-aminophe-nyl)-propan, 4,4',4''-Triaminotriphenylmethan, 4,4'-Diaminodiphenylether, 4,4'4''-Triaminotriphenylthiphosphat, p-Methoxcyanilin, p-Ethoxyanilin, 1-(4-Clorphenoxy)-4-aminobenzol, 2,4-Diaminophenyl-ether, m-Aminobenzoesäureester, p-Aminobenzoesäureester, 3,5-Diamino-2-methyl-diphenylmethan, 3,5-Diamino-4-methyl-diphenylmethan (und Gemische), 3,5-Diamino-4-methyl-dicyclohexylmethan, 3,5-Diamino-2-methyldicyclohexylmethan (und Gemische), 3,5,4'-Triamino-4-methyl-diphenylmethan, 3,5,4'-Tria-mino-2-methyl-diphenylmethan, 3,5,2'-Triamino-4-methyl-diphenylmethan, 3,5,2'-Triamino-2-methyl-diphenylmethan (und Gemische), 3,5,4'-Triamino-4-methyl-dicyclohexylmethan, 3,5,4'-Triamino-2-methyl diclycohexanylmethan, 3,5,4'-Triamino-4-methyl-dicyclohecylmethan, 3,5,2'-Triamino-2-methyl-dicyclohexylmethan (und Gemische), Dibenzofuranamin, 1-Aziridinpropanamin, 4-Pyridinmethanamin, 2-Pyridinamin, 1-3(3-Aminophenyl)-3-methyl-5-pyrazolon, Pyrimidinamin, n-Aminomorpholin, 2-Aminobenzthiazol.

Besonders bevorzugtre Amine sind Propylamin, Isopropylamin, n-Butylamin, sec.-Butylamin, tert.-Butylamin, Stearylamin, Hexamethylendiamin, Cycklohexylamin, 3,3,5-Trimethyl-5-aminomethyl-cyclohe-xylamin, 4,4'-Diaminodicyclohexylmethan, Anilin, p-Chloranilin, 3,4-Dichloranilin, m-Tolylamin, p-Methoxyanilin, 2,4-Diaminotoluol, 2,6-Diaminotoluol, 4,4'-Diaminophenylmethan, 2,4'-Diaminophe-nylmethan oder technische Gemische der gennanten Diaminotoluole bzw. Diaminodiphenylmethane.

Weitere Ausgangsverbindungen für das erfindungsmemäße Verfahren sind

c) Alkohole der Formel

$R_2$-OH

in welcher

$R_2$ die bereits gennante Bedeutung hat.

Beispiele geeigneter Alkohole sind Methanol, Ethanol, Propanol iso-Propanol, Butanol, iso-Butanol, Pentanol, iso-Pentanol, Hexanol, iso-Hexanol, Heptanol, iso-Heptanol, Octanol, iso-Octanol, Nonanol, iso-Nonanol, Decanol, iso-Decanol, Dodecanol, 2-Ethylhexanol, ß-Chlorethanol, 2-Ethylbutanol, Hexadecanol, Octadecanol, Fettalkohol-Gemische, 2-Methoxyethanol, 2-Ethoxyethanol, 2-Propoxyethanol, 2-Butoxyethanol, 2-(2-Methoxyethoxy)-ethanol, 2-(2-Ethoxy)-ethanol, 2-(2-Butoxethoxy)-ethanol, Cyclopentanol, Cyclohexanol, Methylcyclohexanol (und Gemisch), Cyclohexamethanol, 3,3,5-tri-methylcyclohexanol, 4-tert.-Butylcyclohexanol, 2-Hydroxydecalin, Borneol, 1-(2-Hydroxy-ethoxy)-4-nitrobenzol, Benzylankohol, 2-Phenylethanol, 2-(Methoxyphenoxy)-etahnol (Gemisch), 1-Phe-nylethanol, 3-Phenyl-1-propanol, 4-Methoxybenylalkohol.

Besonders bevorzugte Alkohole sind Methanol, Ethanol, n-Propanol, iso-Propanol, n-Butanol, iso-Butanol, Cyclohexanol, n-Hexanol, 2-Ethylhexanol, ß-Phenylethanol, Glykolmonoethylether, Glykolmonobuthylether oder Diglykolmonoethylether.

Erfindungswesentliches Kennzeichen ist nun die Miterwendung weiterer. Carbonylgruppen aufweisender Aufbaukomponenten. Bei diesen weiteren Aufbaukomponenten handelt es sich um

d) N-unsubstituierte Urethane und/oder

e) N-mono- oder N,N'-disubstituierte Harnstoffe bzw. Polyharnstoffe.

Die N-unsubstituierten Urethane, d.h. einfache Carbamate d) entsprechen der Formel

$R_3$-O-CO-$NH_2$

in welcher

$R_3$ der Definition von $R_3$ entspricht, wobei $R_3$ und $R_2$ für gleiche oder verschiedene Reste, vorzugweise jedoch für gleiche Reste stehen, Außerdem kann $R_3$ auch für einen gegebenenfalls Chloroder $C_1$-$C_4$-Alkyl-substituierten aromatischen Kohlenwasserstoffrest mit insgesamt 6 bis 15 Kohlenstoffatomen stehen.

Typische Beispiele geeigneter N-unsubstituierter Urethane d) sind demzulfolge Methyl-, Ethyl, n-Prpoyl-, iso-Propyl), n-Butyl-, iso-Butyl-, Cyclohexyl- oder n-Hexylcarbamat oder die sich aus den sonstigen oben beispielhaft aufgezählten Alkoholen ableitenden Carbamate oder auch Phenyl-, 2-Chlorophenyl-, 3-Chlorophenyl-, 4-Chlorophenyl-, 2-Methylphenyl-, 4-Methylphenyl-, 4-n-Butylphenyl- oder 1-Naphthyl-Carbamat.

Bei der Komponente e) handelt es sich um organische, gegebenenfalls Urethan- oder primäre Aminoendgruppen auweisende N-mono- oder N,N'-disubstituierte Harnstoffe oder lineare Polyharnstoffe eines maximalen Molekulargewichts von 2 000 vorzugweise 700, deren Harnstoff-, Urethan- bzw. Aminogruppen über Kohlenwasserstoffreste miteinander verknüpft sind, bzw. deren Harnstoffgruppen mit Kohlenwasserstoffresten substituiert sind, die vorzugweise dem Kohlenwasserstoffrest des eingesetzten Amins b) entsprechen und deren gegenbenenfalls vorliegenden endständigen Urethangruppen am Sauersoffatom mit Kohlenwasserstoffresten substituiert sind, die vorzugsweise dem Kohlenwasserstoffrest des Alkohols c) entsprechen. Typische Beispiele geeigneter Harnstoffe bzw. Polyharnstoffe e) sind N-Methylharstoff. N-Ethylharnstoff. N-(n-Propyl)-harnstoff, N-(iso-Propyl)-harnstoff, N-(n-Butyl)-harnstoff, N-(iso-Butyl)-harnstoff, N-Cyclohexylharnstoff, N-Benzylharnstoff, N,N'-Dimethylharnstoff, N,N'-Diethylharnstoff. N,N'-Di-(n-Butyl)-harnstoff, N,N'-Dicyclohexyl-harnstoff, N,N'-Di-Benzyl-harnstoff, N, N'-Di-(m-tolyl)-harnstoff, N-Phenylharnstoff, N,N'-Diphenylharnstoff, N,N'-Di-Carbamoyl-toluylendiamin-2,4, N,N'-Di Carbamoylsiophronodiamin oder Verbindungen der Formel

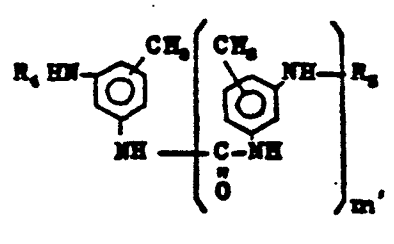

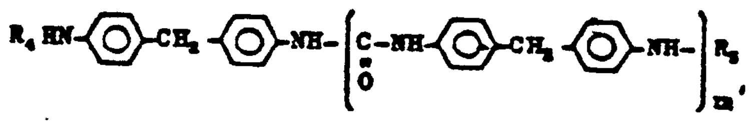

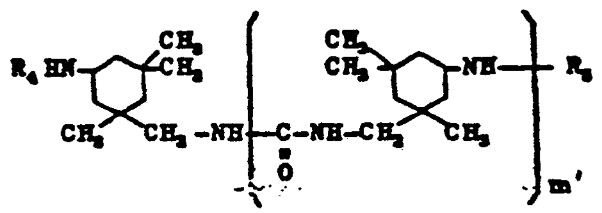

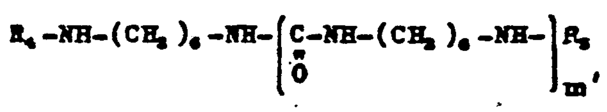

wobei m' eine ganze oder gebrochenen (bei statistischen Gemischen) Zahl von 1 bis 10 ist.

$R_4$ und $R_5$ für gleich oder verschiedene Reste stehen und jewils H, $COOR_2$, $CONH_2$, $CONHR_6$ bedeuten, wobei

$R_6$ für eien einwertigen Rest der bei der Definiton vn $R_1$ genannten Art steht.

Die entprechenden Diharnstoffe bzw. die beispielhaft gennanten N,N'-disubstituierten einfachen Harnstoffe weden bevorzugt verwendet.

Geeignete Harnstoffe sind beispielweise auch die N,N'-disubstituierten Harnstoffe bzw. die entsprechenden Polyharnstoffe, die als Nebenprodukte bei in den US-Patentachriften 2 409 712 und 2 806 051 angegeben Verfahren anfallen. Es können aber auch nach anderen Wegen sunthetisierte entsprechend substituierte Harnstoffe (D.F. Kutepow. Russ. Chem. Rev. 31, 633 (1962) oder Polyharnstoffe (H. Rinke, Houben-Weyl XIV/2, 165 ff) eingesetzt werden.

Bei der Durchführung des erfindungsgemäßen Verfahrens kommt die Amin-Komponente b) inklusive dem in der Koponente e) chemisch gebunden Amin, falls dieses der Amin-komponente b) entpricht, in einer 0,5-4 fach, vorzugsweise 0,88-1,5-fach und insbesondre 0,9-1,1-fach stöchiometrischen Menge und die Alkohol-Komponente c) inklusive dem in den Komponente d) und e) chemisch gebunden Alkohol, falls dieser der Alkohol-Komponente c) entspricht, in einer 1-10-fach, vorzugsweise 1,1-4-fach stöhiometrischen Menge, jeweils bezogen auf die in den Komponenten a), d) und e) innerhalb von Harnstoff-, Polyuretoder Urethangruppen vorliegenden Carbonylgruppen zum Einsatz. Die Komponenten d) und/oder e) werden im allgemeinen jeweils in einer Menge von 0 bis 300, vorzugsweise 0 bis 150 Gew.-%, bezogen auf die Mengen des Harnstoffs oder der Polyurete a) eingesetzt, mit der Einschränkung, das die Gesamtmenge der Komponenten d) und e) mindestens 10, vorzugweise mindestens 30 Gew.-% beträgt.

Das erfindungsgemäße Verfahren wird vorzugsweise unter Mitverwendung von Katalysatoren durchgefürt. Geeignete Katalysatoren sind alle beliebigen Verbindungen, die einen katalytischen Einfkuß auf die Veresterungsschwindigkeit von Carbonsäuren aufweisen. Besonders gut geeignete Katalysatoren sind (I) unter den Reaktionsbedingungen inerte anorganische oder organische Basen, (II) Lewis-Säuren und (III) Salze bzw. Komplexverbindungen, insbesondere Chelate von Übergangsmaterialien.

Beispiele geeigneter Katalysator der Gruppe (I) sind tert.-Amine wie Tri-n-propylamin, Triethylamin, Triisopentylamin, Diethylbenzylamin, N,N-Dimethyl-benzylamin, Hexahydridimethylanilin, N-Ethylpoperazin, Diethyl(2-methoxypropyl)-amin, 2-(Diethylammoethyl)-phenylether, N-(2-Diethylami-noethyl)-benzamid, N-(2-Diethylaminoethyl)-propionamid, 1,4-Diaza-(2,2,2)-bicyclooctan, N,N-Diemethyl-4-aminopyridin, 1-Azabicycloheptane, 1-Azabicyclooctane, gesättige polyhetercyclische Amine, wie 3-Methylconidin, 1-Azabicyclo-(3,2,1)-octan, Pyrrolozidine und Chinuclidine anorganische Basen wie Beryliumhydroxid und Natrium-, Kalium-, Lithium-, Magnesium-, Barium- oder Calciumhydroxid, basische Alkalisalze wie Natriumcarbonat, Natriumsulfid, Kaliumcarbonat oder Trinatriumphosphat sowie Alkalisesalze von Fettsäuren oder Sulfonsäuren.

Geeignete Katalysatoren (III) sind beispielsweise Lewis-Säuren wie Eisen-II-cloridm Eisen-III-chlorid, Zinkchlorid, Zinn-II-chlorid, Zinn-IV-chlorid, Aluminiumchlorid, Zinkcyanid, Bortrifluorid oder Bortrifluoredietherat.

Geeignete Katalysatoren der Gruppe (III) sind beispielweise Salze von Übergangsmetallen, soweit sie nicht bereits in die Gruppe (II) fallen, sowie Komplexverbindungen insorbendere Chelate dieser Metalle wie Kobalt-, Mangan-, oder Bleinaphthenate, Eisenoleate oder -carbonyle, Acetylacetonate von Eisen, Nickel, Kobalt, Zink, Blei Aluminium, Mangan, Magnesium, Molybdän, Titan, Torium Zirkon oder Vanadium, Bis-(disbenzolymmethan)-kupfer, -eisen, Koordinationsverbindungen von Titan, Zirkon, Hafnium, Thorium und Xangan mit ß-Diktetonen, ß-Ketoestern und ß-Hydroxyaldehyden, Dibutylzinndilaurat, Dibutylzinndiacetat, Di-(2-Ethyl)-zinnoxid, Dioctylzinnoxid, Zink oder Zinnsalze von $C_1$-$C_{20}$-Carbonsäuren wie Zink- oder Zinn (II) naphthenat, -hexoat, -calmitat, -hexoat, -calmitat, -stearat oder dimethylvelerat, Acetate, Chloride, Sulfate oder Octoate des zwei- oder dreiwerigen Cobalts, des ein- oder zweiwertigen Kupfers oder des zweiwertigen Bleis.

Besonders gut geeignete Katalysatoren sind beispielweise Zinkchlorid, Zinkacetat, Zinkoctoat, Zinkoxid, Zinkcyanid, Zinn-II-chlorid, Dibutylzinndilaurat, Kobalttriacetat, Kobaltrichlorid. Kobaltttrioctoat, Kupfer (II)-acetat, Kupfer-(I)-chlorid, Kupfer-(II)-sulfat, Bleiacetat oder Bleichlorid. Die Menge des jeweils eingesetzen Katalysators liegt im allgemeinen zweischen 1 ppm und 20 Gew.-%, bevorzugt zwischen 100 ppm und 5 Gew.-%, bezogen auf die Summe der Ausgangsmaterialen a), b), c), d) und e). Man wird in der Praxis selbstverständlich bestebt bleiben, die Konzentration der Katalysatoren möglichst gering zu halten. Die optimale Konzentration hängt von der Art der Ausgangsmaterialien und der Aktivität des jeweiligen Katalysators ab und kann in einem enfachen Vorversuch bestimmt werden.

Die Durchführung des erfindungsgemäßen Verfahren kann sowohl unter Druck als auch drucklos erfolgen. Die Anwendung von Druck beispielweise im Bereich von 1 bis 80 bar ist oft dann sinnvoll, wenn die Reaktionstemperatur oberhalb dem Siedepunkt einer oder mehrer der Ausgangsmeterialien liegt. Das erfindungsgemäße Verfahren wird im Temperaturbereich von 120°C bis 350°C, vorzugsweise 130 bis 300°C und insbesondere 140°C bis 250°C durchgeführt.

Das erfindungsgemäße verfahren kann in Gegenwart oder auch in Abwesenheit von Lösungsmitteln durchgeführt werden, Geeignete Lösungsmittel sind beispielsweise unter den Verfahrensbedingungen inerete Lösungsmittel mit einem zwischen 100°C und 280°C, vorzugweise zwischen 150°C und 250°C liegenden Siedepunkt. Beispiele geeigneter Lösungsmittel sind n-Nonan, n-Butylcyclohexdan, Decahydronaphtahlin, n-Undecan, n-Dodecan, n-Hexylcyclohexan, Dipenten, 1-Dodecen, Isopropylbenzol, 1,3-Diethylbenzol, Inden, n-Butylbenzol, Tetralin, Chlorbenzol, 4-Chlortoluol, 1,2-Dichlorbenzol, 2,4-Dichlortoluol, 1,2,4-Trichlorbenzol, 2-Chlor-4-isopropyl-1-methylbenzol. Anisol, Cyclohexylethylether, Diethylenglykoldimethylethr, Benzylmethylether, 4-Methoxyloluol, Parachloranisol, Di-n-hexylether, Phenyl-n-propylketon, Benzophenon, Acetophenon, Formamid, N,N-Dimethylformamid, N,N-Diethylformamid, N-Methylformamid, Dimethylacetamid, N-Methylpryrrolidon, Caprolactan, Phenol, substituirte Phenole, Sulfolan, Hexamethylphosphosäuretriamid, Dimethylsulfoxid, Ethylglykolmonomethyletheracetat, Di-n-propylcarbonat, Cyclohexylacetat, Diisobutylcarbonat, Diethylenglykolmonomethyleteracetat, Diisoamyldarbonat, 2-Ethylpyridin, N,N-Dimethyl-2-methylanilin, N,N-Dimethylanilin, N-Methyl-N-ethylanilin, N,N-Dimethyl-2-chloranilin, N,N-Diethylanilin, Chinolin, Nitrocyclohexan, Nitrobenzol, 2-Nitrotoluol, 2,4-Dimethyl-1-nitrobenzol, Acetonfitril, N-Capronitril, Benzolnitril, Tolunitril, Diphenyläther, Tetramethylhanrstoff und Phenylacetonitril, besonders bevorzugt sind polare Lösungsmittel und deren Gemische. Zu den ganz besonders gut geeigneten Lösungsmitteln gehört ε-Caprolactam.

Die Mitverwendung deratiger Lösungsmittel ist oft, beispielweise bei Verwendung eines hohen Überschusses an Alkohol (Komponente c) überflüssig. Insbesondere erübrigt sich die Verwendung deratiger Hilfslösungsmittel bei der Herstellung Monourethanen (n=1), d.h. bei der ausschließlichen Verwendung von Monaminen als Komponente b).

Da die erfindungsgemäße Umsetzung zur Abspaltung von Ammoniak abläuft, ist auch bei der Druckreaktion gegenbenenfalls für dessen Entfernung zu sorgen. Dies kann durch Einbau geeigneter Überdruckventile in die Druckapparatur geschehen.

Auch bei Verwendung von niedrig siedenden Alkoholen ist man nicht unbedingt auf eine Durchführung der Umsetzung unter Druck angewiesen, da man auch in der Weise vorgehen kann. daß man die Reaktionsparther a), b), d) bzw. e) auf die Reaktionstemperatur erhitzt und den niedrig siedenden Alkohol so langsam zugibt, daß die Reaktionstemperatur erhalten bleibt.

Zur Durchführung des erfindungsgemäßen Verfahrens werden Reaktionspartner gemischt und auf die angestrebte Reaktionstemperatur erhitzt. Bei Verwendung von Alkoholen c) die einen höheren Siedepunkt aufweisen als die Alkoholkomponente der gegebenenfalls eingesetzten Komponente d) und die gegebenenfalls in der gegebenenfalls eingesetzten Komponente e) vorliegende Alkoholkomponente werden im allgemeinen die letztgenannten Alkoholkomponenten bei der Durchführung des ertindungsgemäße Verfahrens verdrängt, so daß im Verfahrensprodukt ausschließich die höhersiedende Alkoholkomponente c) vorliegt. Dies ist auch bei Verwendung von aromatischen N 1 unsubstituierten Urethanen d) der Fall, ohne daß

es in diesem letztgenannten Fall erforderlich ware, die Hydroxylkomponente des Urethans d) destillativ aus dem Gleichgewicht zu entfernen.

Das erfindungsgemäße Verfahren läuft, wie nachstehend am Beispiel einiger einfacher Ausgangsmaterialien a)e) verdeutlicht, im Idealfall unter Einbau aller Ausgangsmaterialien in das Verfahrensprodukt ab.

1.

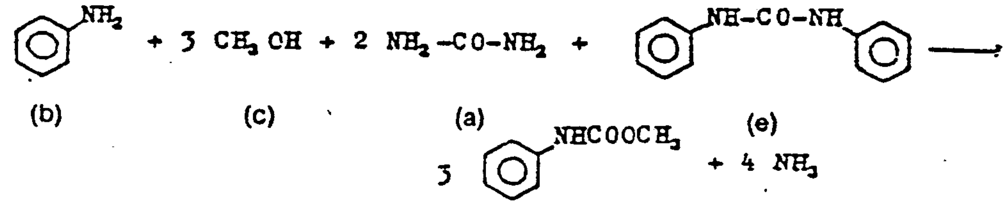

(b) (c) (a) (e)

2.

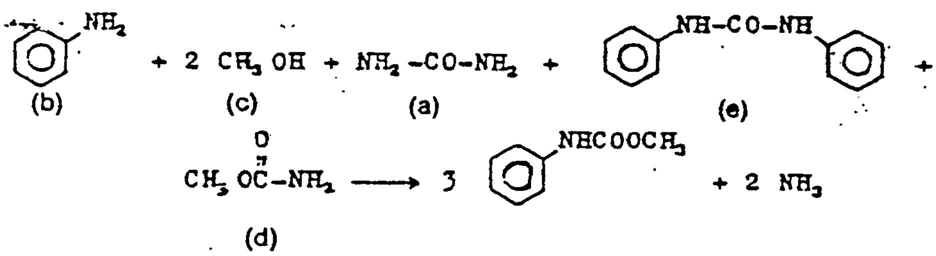

(b) (c) (a) (e) (d)

In der Praxis wird jedoch das Verfahrensprodukt im allgemeinen noch untergeordnete Mengen an Ausgangsmaterialien d) bzw. e) enthalten. Es ist selbstverständlich möglich, diese nicht völlig umgesetzten Ausgangsmaterialien vom Verfahrensprodukt abzutrennen und wieder zu verwenden.

Die erfindungsgemäße Umsetzung ist im allgemeinen nach einer Zeitspanne von 2 bis 15, insbesondere 2 bis 12 Stunden beendet. Die Aufarbeitung des Reaktionsgemisches erfolgt in an sich bekannter Weise, insbesondere durch Abdestillieren leicht flüchtiger Hilfsmittel bzw. leicht flüchtiger überschüssiger Ausgangsmaterialien, gegebenfalls nach Abfiltrieren unlöslicher Bestandteile wie z. B. unlöslicher Katalysatoren. Die Verfahrensprodukte fallen bei der destillativen Aufarbeitung im allgemeinen als letzte Fraktion bzw. als Destillationsrückstand an. Die Abtrennung von gegebenenfalls in dem so erhaltenen Verfahrensprodukt enthaltenem substituiertem Harnstoff e) kann beispielsweise durch Aufnahme des Destillationsrückstands in einem geeigneten selektiven Lösungsmittel wie z. B. Testbenzin und anschließendes Abfiltrieren, des Harnstoffs e) erfolgen.

Das erfindungsgemäße Verfahren gestattet eine Erhöhung der Ausbeute an N,O-disubstituiertem Urethan, bezogen auf eingesetzten Harnstoff. Die Tatsache, daß dies durch die erfindungsgemäße Maßnahme der Mitverwendung der Komponenten d) und/oder e) möglich ist, muß als überraschend bezeichnet werden, da gerade diese Komponenten beim bekannten Verfahren der Urethansynthese aus Harnstoff. Amin und Alkohol gemäß US-Patenten 2409712 oder 2806 051 unerwünschte Nebensprodukte darstellen, die zu einer vergleichsweisen schlechten Ausbeute der Verfahren dieser Vorveröffentlichungen führen.

Die erfindungsgemäßen Verfahrensprodukte stellen wertvolle Ausgangsmaterialien zur Herstellung der ihnen zugrundeliegenden Isocyanate dar. Diese Herstellung von organischen Isocyanaten unter Verwendung der erfindungsgemäßen Verfahrensprodukte geschieht durch deren ansich bekannte thermische Spaltung in das ihnen zugrundeliegende Isocyanat und den ihnen zugrundeliegenden Alkohol. Es ist im allgemeinen nicht erforderlich, die erfindungsgemäßen Verfahrensprodukte vor ihrer Verwendung zu reinigen d. h. insbesondere sie von anhaftenden Ausgangsmaterialien d) und e) zu befreien.

Die in den nachfolgenden Beispielen emähnten Prozentangaben beziehen sich auf Gewichtsprozente.

**Beispiel 1**

Zur Umsetzung von niedrigsiedenden Produkten wird eine Druckapparatur verwendet. Sie besteht aus einem Druckbehälter (Stahl, Nenninhalt 5 l, zulässiger Höchstdruck 64 bar) mit Rührer und Mantelheizung und einer Füllkörperkolonne (Stahl, Nennweite 50 mm). Der Druckbehälter dient sowohl als Reaktionsgefäß als auch als Sumpfgefäß für die Kolonne. Die Kolonne ist ca. 1 m hoch mit Füllringen aus Stahlmaschendrahtgewebe (4 mm) gefüllt. Sie ist mit einem Schlangenkühler als Kopfkondensator versehen, oberhalb dessen sich ein Ventil zur Entnahme von Gasen befindet.

In den Druckbehälter werden 745 g Anilin 849 g N,N'-Diphenylharnstoff: 480 g Harnstoff, 356g Ethylcarbamat und 1 350 g Ethanol (ca. 96%ig) unter Ammoniak-Entnahme 5,5 Stunden bei 200 °C umgesetzt. Nach dem Abkühlen und Entspannen der Apparatur wird das Reaktionsgemisch entnommen, filtiert und fraktioniert

destilliert. Nachdem bei Normaldruck überschüssiger Alkohol abgetrennt ist, wird bei der Destillation bei 0,2 mbar ain Hauptlauf von 1 934 g (73,2% der Theorie) N-Phenyl-carbamidsäureethylester vom Schmelzpunkt 49-51 °C erhalten.

**Beispiel 2**

In die im Beispiel 1 beschriebene Apparatur werden 169 g eines Polyharnstoff-Gemisches auf Basis von 2,4-Diaminotoluol mit Aminotolyl-Endgruppen (mittleres Molgewicht: 1 500) eingefült. Dann werden 428 g 2,4-Diamino-toluol,384 g Harnstoff, 168 g Ethylcarbamat. 1 700 g Ethanol (ca. 96%ig) und 5,2 g Zinkoctoat dazugegeben. Das Gemisch wird analog zu Beispiel 1 unter Ammoniak-Entnahme 6,0 Stunden bei 200 °C umgesetzt. Nach dem Abkühlen und Entspannen der Apparatur wird das Reaktionsgemisch entnommen, filtriert und mit Hilfe der Hochdrucktlüssigkeitschromatographie analysiert. Nach Eichung mit einer authentischen Substanz werden 937 g 2,4-Bis-(ethoxycarbonylamino) toluol ausgewiesen; das entspricht einer Ausbeute von 75% der Theorie.

**Beispiel 3**

102,3 g (1.1 Mol) Anilin, 300 g (3 Mol) Cyclohexanol, 60 g (1 Mol) Harnstoff. 136,2 g (1 Mol) Phenylharnstoff und 3 g Cobaltnaphthenat wurden 3 Stunden am Rücktluß erhitzt. Nachdem 200 °C Sumpftemperatur erreicht waren, wurde nochmals 4 Stunden bei dieser Temperatur gehalten.

Mit Hilfe der Hochdruckflüssigkeitschromatographie (HPLC) wurde die Ausbeute an O-Cyclohexyl-Nphenylurethan zu 93 % der Theorie bestimmt.

**Beispiel 4**

109 g (1,1 Mol) Cyclohexylamin, 90 g (1,5 Mol) Harnstoff, 366 g (3 Mol) β-Phenylethanol und 112 g (0,5 Mol) N,N'-Dicyclohexylharnstoff wurden 10 Stunden bei 200°C erhitzt. Nach Einengen wurde der in Essigsäureethylester unlösliche Teil abgetrennt und 439 g = 89% der Theorie an N-Cyclohexyl-O-βphenylethylurethan vom Schmelzpunkt 93 °C (Waschbenzin) isoliert.

**Beispiel 5**

102,3 g (1,1 Mol) Anilin, 300 g (3 Mol) Cyclohexanol 51,5 g (0.5 Mol) Biuret und 136,2 g Phenylharnstoff wurden 16 Stunden am Rückfluß erhitzt. Die Ausbeute an O-Cyclohexy-N-phenylurethan wurde hochdruckflüssigkeitschromatographisch zu 71% der Theorie bestimmt.

Obige Reaktion wurde mit 0,7 g Zinkoctoat wiederholt, wobei jedoch nur 9 Stunden am Rückfluß erhitzt wurde. Die Ausbeute an O-Cyclohexyl-N-phenylurethan wurde hochdruckflüssig keitschromatographisch zu 96% der Theorie bestimmt.

**Beispiel 6**

204,6 g (2,2 Mol) Anilin, 300 g (3 Mol) Cyclohexanol, 96 g (1.6 Mol) Harnstoff und 54,4 g (0.4 Mol) Carbamidsäurephenylester wurden mit 1 g® Dabco am Rückfluß bis auf 200 °C erhitzt und dort 7 Stunden gehalten. Mit Hilfe der Hochdruckflüssigkeitschromatographie wurde die Ausbeute an O-Cyclohexyl-Nphenylurethan zu 87% der Theorie bestimmt.

**Beispiel 7**

93 g (1 Mol) Anilin, 90 g (1,5 Mol) Harnstoff, 106 g (0,5 Mol) N,N'-Diphenylharnstoff und 427 g (3. 5 Mol) β-phenylethanol wurden innerhalb von 2 Stunden auf 200 °C erhitzt. Nach weiteren 7 Stunden bei 200 °C wurde mit Hilfe der Hochdrucktlüssigkeitschromatographie eine Ausbeute von 88% der Theorie an N-Phenyl-O-β-phenylethylurethan gefunden.

**Beispiele 8**

212 g N,N'-Diphenylharnstoff (1 Mol), 75 g Carbamidsäuremethylester (1 Mol), 93 g Anilin (1 Mol). 60 g Harnstoff (1 Mol) und 400 g Cyclohexanol (4 Mol) werden mit 6 ml Zinkoctoat versetzt und innerhalb von 3 Stunden auf 200°C erwärmt. Das entstehende Methanol wird während der Umsetzung abdestilliert. Es wird weitere 4 Stunden bei 200 °C gehalten. Anschließend werden 90 g Cyclohexanol im Wasserstrahlvakuum abdestilliert, der Rückstand (665 g) besteht zu 96,5% aus N-Phenyl-O-cyclohexylurethan (bestimmt durch HPLC).

Fp: 81-82 °C (aus Waschbenzin).

**Beispiel 9**

93 g Anilin (1 Mol), 60 g Harnstoff (1 Mol), 212 g N,N'-Diphenylharnstoff (1 Mol). 143 g Cyclohexylcarbamat (f Mol) und 270 g Cyclohexanol (2,7 Mol) werden mit 3 ml Zinkoctoat versetzt und auf 150 °C erwärmt. Die Innentemperatur steigt innerhalb von 4 Stunden auf 190 °C an. Dann wird weitere 5 Stunden bei 200 °C gerührt. Nach dem Ende der Umsetzung wird im Wasserstrahlvakuum das noch vorhandene Cyclohexanol abdestilliert.

Der Rückstand (660 g) besteht zu 96% aus N-Phenyl-0-cyclohexylurethan (bestimmt durch HPLC).

Fp: 81-82 °C (aus Waschbenzin).

**Beispiel 10**

240 g 3,3'-Dimethyl-N,N'-diphenylharnstoff (1 Mol). 107 g 3-Aminotoluol (1 Mol), 120 g Harnstoff (2 Mol) und 488 g 2-Phenylethanol (4 Mol) werden nach Zugabe von 2 g Aluminiumchlorid zügig bis zur beginnenden $NH_3$-Entwicklung erwärmt. Dann die Temperatur innerhalb von 2 Stunden auf 200 °C gesteigert und 6 Stunden bei dieser Temperatur gehalten.

Danach wird an der Olpumpe nicht umgesetztes 2-Phenylethanol abdestilliert.

Ausbeute: 628 g N-(m-Tolyl-O-2-phenylethyl)-urethan (= 82% der Theorie).

Fp: 52-53 °C (aus Petrolether).

**Beispiel 11**

9,3 kg Anilin (100 Mol), 6 kg Harnstoff (100 Mol), 21,2 kg N,N'-Diphenylharnstoff (100 Mol), 143 kg Cyclohexylcarbamat (100 Mol) und 26 kg Cyclohexanol (260 Mol) werden mit 200 ml Zinkootvat versetzt und auf 155°C erwärmt. Die Innentemperatur steigt innerhalb von 5 Stunden auf 19O°C, dann innerhalb einer weiteren Stunde auf 200 °C. Bei dieser Temperatur wird 5 Stunden nachgerührt. Nach der Umsetzung wird überschüssiges Cyclohexanol im Wasserstrahlvakuum abdestilliert. Nach HPLC besteht der Rückstand zu 95% aus N-Phenyl-cyclohexylurethan. Aus dem Rückstand werden 260 · g Cyclohexylcarbamat durch Vakuumdestillation entfernt. Der so erhaltene Rückstand wird in 100 Liter heißem Waschbenzin ausgenommen und anschließend filtriert. Auf dem Filter hinterbleiben nach dem Trocknen 638 g Diphenylharnstoff. Die Waschbenzinlösung wird eingeengt und auf diese Weise ein von Nebenprodukten weitgestgehend befreites N-Phenyl-cyclourethan erhalten.

Ausbeute: 61,76 kg N-Phenyl-O-cyclohexylurethan (= 94% der Theorie).

Fp: 81-B2°C (aus Waschbenzin).

In einem zweiten Ansatz, wie zuvor beschrieben. werden statt 14,3 kg reines Cyclohexylcarbamat (100 Mol) nur 14,0-1 kg Cyclohexylcarbamat und 260 g aus dem ersten Ansatz zurückgewonnenes Cyclohexylcarbamat verwendet. Es wird in gleicher Weise wie zuvor beschrieben gearbeitet.

Ausbeute: 61.8 kg N-Phenyl-O-cyclohexylurethan (= 94% der Theorie).

In einem dritten Ansatz werden statt 21,2 kg frischen N,N'-Diphenylharnstoffs nur 20,6 kg verwendet, dafür 600 g zurückgewonnenen N,N'-Diphenylharnstoff aus dem zweiten Ansatz mitverwendet. Ansonsten werden Anilin. Harnstoff, Cyclohexylcarbamat, Cyclohexanol und Zinkoctoat wie im ersten Ansatz beschrieben. umgesetzt.

Ausbeute: 61,78 kg N-Phenyl-O-cyclohexylurethan (= 94% der Theorie).

Fp: 81-82°C (aus Waschbenzin).

In einem vierten Ansatz werden 1,2 kg N,N'-Diphenylharnstoff und 500 g Cyclohexylcarbamat aus den Ansätzen eins bis drei mitverwendet. Die Umsetzung wird wie im ersten Ansatz durchgefü hrt. Die Einsatzmengen an reinem N,N'-Diphenylharnstoff und reinen Cyclohexylcarbamat werden um die zurückgewonnenen Beträge verringert.

Ausbeute: 61,85 kg N-Phenyl-O-cyclohexylurethan (= 95% der Theorie).

Fp: 82°C (aus Waschbenzin).

**Patentansprüche**

1. Verfahren zur Herstellung von Urethanen der allgemeinen Formel

$$R_1\left[NH-\overset{\overset{O}{\|}}{C}-O-R_2\right]_n$$

in welcher

$R_1$ für einen gegebenenfalls substituierten aliphatischen Kohlenwasserstoffrest mit 1-18 Kohlenstoffatomen, einen gegebenenfalls substituierten cycloaliphatischen Kohlenwasserstoffrest mit 3 bis 18 Kohlenstoffatomen, einen gegebenenfalls substituierten aromatischen Kohlenwasserstoffrest mit 6-15 Kohlenstoffatomen, einen gegebenenfalls substituierten araliphatischen Kohlenwasserstoffrest mit 7 bis 14 Kohlenstoffatomen, oder für einen gegebenenfalls substituierten 5- oder 6-gliedrigen heterocyclischen Rest steht. der zusätzlich noch mit einem Benzolring anelliert sein kann,

$R_2$ für einen gegebenenfalls substituierten Alkylrest mit 1 bis 20 Kohlenstoffatomen, einen gegebenenfalls substituierten Cycloalkylrest mit 3 bis 16 Kohlenstoffatomen und einen gegebenenfalls substituierten Aralkylrest mit 7 bis 14 Kohlenstoffatomen und

n eine ganze Zahl von 1 bis 3 bedeuten. wobei im Falle von n = 2 oder 3 wischen 2 mit dem Rest $R_1$ verknüpften U rethangruppen mindestens 2 Kohlenstoffatome angeordnet sind durch Umsetzung von

a) Harnstoff oder Polyurethan der Formel

$$H_2N—(CO—NH)_m—H$$

in welcher

m für eine ganze Zahl von 1 bis 4 steht, bzw. Gemische derartiger Verbindungen, mit

b) primären Aminen der Formel

$$R_1(NH_2)_n$$

in welcher

$R_1$ und n die bereits genannte Bedeutung haben, und

c) Alkoholen der Formel

$$R_2—OH$$

in welcher

$R_2$ die bereits genannte Bedeutu ng hat, im Temperaturbereich von 120 bis 350 °C. dadurch gekennzeichnet, daß man als weitere Reaktionspartner

d) N-unsubstituierte Urethane der Formel

$$R_3—O—CO—NH_2$$

in welcher

$R_3$ der Definition von $R_2$ entspricht. wobei Ra und $R_2$ für gleiche oder verschiedene Reste stehen, oder in welcher $R_3$ für einen gegebenenfalls Chlor oder $C_1$-$C_4$-Alkyl-substituierten aromatischen Kohlenwasserstoffrest mit insgesamt 6 bis 15 Kohlenstoffatomen steht, und/oder

e) gegebenenfalls Urethan- oder primäre Aminoendgruppen aufweisende N-mono- oder N,N'disubstituierte Harnstoffe oder lineare Polyharnstoffe eines maximalen Molekulargewichts von 2 000, deren Harnstoff-Urethan- bzw. Aminogruppen über Kohlenwasserstoffreste miteinander verknüpft sind, bzw, deren

Harnstoffgruppen mit Kohlenwasserstoffresten substituiert sind, die dem Rest $R_1$ entsprechen, und deren gegebenenfalls vorliegende endständige Urethangruppen am Sauerstoffatom mit Kohlenwasserstoffresten substituiert sind, die dem Rest $R_2$ entsprechen, mitverwendet, wobei die Herstellung von Arylmono-, di- und/oder -polyurethanen durch Umsetzung von gegebenenfalls substituierten O-Alkyl-, O-Cycloalkyl- oder O-Aralkylcarbamidsäureestern mit primären aromatischen Mono-, Di- und/oder Polyaminen in Gegenwart von Alkoholen und Harnstoff ohne gleichzeitige Mitverwendung der unter el oenannten Ausqangsmaterialien ausgenommen ist wobei die Reaktionspartner a) bis e) zur Durchführung des Verfahrens mitejnander gemischt und auf die Reaktionstemperatur von 120° C bis 350°C erhitzt werden, und wobei die Amin-Komponente b), inklusive dem in der Komponente e) chemisch gebundenen, der Amin-Komponente b) entsprechenden Amin, in einer 0,5-bis 4-fach stöchiometrischen Menge und die Alkohol-Komponente c) inklusive dem in den Komponenten d) und e) chemisch gebundenen Alkohol, falls dieser der Alkohol-Komponente c) entspricht, in einer 1-la-fach stöchiometrischen Menge, jeweils bezogen auf die in den Komponenten a), d) und e) vorliegenden Carbonylgruppen und die Komponenten d) und e) in einer Menge von 0 bis 300 Gew.-%, bezogen auf die Menge der Komponente a) eingesetzt werden, mit der Einschränkunq, daß die Gesamtmenge der Komponenten d) und e) mindestens 10 Gew.-% beträgt.

2.Verfahren gemäß Anspruch 1 dadurch gekennzeichnet, daß man die Umsetzung in Gegenwart von Versterungskatalusatoren für Carbonsäuren als Katalysatoren durchführt.

3. Verfahren gemäß Anspruch 1 und 2, dadurch gekennzeichnet, daß man die Umsetzung in Gegenwart von polaren Lösungsmitteln durchführt.

4. Verfahren gemäß Anspruch 3, dadurch gekennzeichnet, daß man ε-Caprolactam als Lösungsmittel verwendet.

## Claims

1. Process for the production of urethanes of the general formula

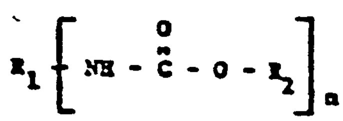

in which

$R_1$ represents an optionally substituted aliphatic. hydrocarbon radical with 1-18 carbon atoms, an optionally substituted cycloaliphatic hydrocarbon radical with 3 to 18 carbon atons, an optionally substituted aromatic hydrocarbon radical with 6-15 carbon atoms, an optionally substituted araliphatic hydrocarbon radical with 7 to 14 carbon atoms, or an optionally substituted 5- or 6-membered heterocyclic radical which can additionally be fused to a benzene ring,

$R_2$ represents an optionally substituted alkyl radical with 1 to 20 carbon atoms, an optionally substituted cycloalkyl radical with 3 to 16 carbon atoms and an optionally substituted aralkyl radical with 7 to 14 carbon atoms and

n denotes an integer from 1 to 3, wherein in the case where n = 2 or 3, at least 2 carbon atoms are arranged between 2 urethane groups attached to the radical $R_1$,

by reacting

a) urea or polyurethane of the formula

$H_2N\text{-}(CO\text{-}NH)_m\text{-}H$

in which

m represents an integer from 1 to 4,

or mixtures of such compounds, with

b) primary amines of the formula

in which

$R_1$ and n have the meaning already mentioned, and

c) alcohols of the formula

$R_2\text{-}OH$

in which

$R_2$ has the meaning already mentioned, in the temperature range of 120 to 350° C, characterised in that

d) N-unsubstituted urethanes of the formula

$R_3\text{-}O\text{-}CO\text{-}NH_2$

in which

$R_3$ corresponds to the definition of $R_2$, $R_3$ and $R_2$ representing identical or different radicals

or in which

$R_3$ represents an optionally chlorine- or $C_1$-$C_4$-alkyl-substituted aromatic hydrocarbon radical with a total of 6

to 15 carbon radicals, and/or

a) N-mono- or N,N'-disubstituted ureas or linear polyureas of a maximum molecular weight of 2,000, which optionally contain urethane or primary amino end groups and whose urea, urethane or amino groups are linked to one another via hydrocarbon radicals, or whose urea groups are substituted by hydrocarbon radicals which correspond to the radical $R_1$, and whose optionally present terminal urethane groups are substituted on the oxygen atom by hydrocarbon radicals corresponding to the radical $R_2$, are also used as further reactants, with the exception of the production of aryl-mono-, -di- and/or -polyurethanes by the reaction of optionally substituted O-alkyl-, O-cycloalkyl- or O-aralkylcarbamic acid esters with primary aromatic mono-, di- and/or polyamines, in the presence of alcohols and urea without the simultaneous usa of the starting materials mentioned under e), wherein the reactants a) to e) are mixed with each other for the execution of the process and heated to a reaction temperature of 120°C to 350°C, and wherein the amine component b), including the amine which corresponds to amine component b) and is chemically bonded in component e), is used in,0.5 to 4 times the stoichiometric amount and the alcohol-component c), including the alcohol chemically bonded in components d) and e), if this alcohol corresponds to the alcohol component c), is used in 1-10 times the stoichiometric amount, in each case based on the carbonyl groups present in components a), d) and e) and the components d) and e) are used in a quantity of 0 to 300% by weight, based on the amount of component a), with the limitation that the total amount of components d) and e) is at least 10% by weight.

2. Process according to Claim 1, characterised in that the reaction is carried out in the presence of esterification catalysts for carboxylic acids-as the catalysts.

3. Process according to Claim 1 and 2, characterised in that the reaction is carried out in the presence of polar solvents.

4. Process according to Claim 3, characterised in that ε-caprolactam is used as the solvent.

**Revendications**

1. Procédé pour la fabrication d'uréthannes de formule générale

$$R_1\left[NH - \overset{O}{\overset{\|}{C}} - O - R_2\right]_n$$

dans laquelle

$R_1$ représente un reste d'hydrocarbure aliphatique en $C_1$-$C_{18}$ éventuellement substitué un reste d'hydrocarbure cycloaliphatique en $C_3$-$C_{18}$ éventuellement substitué un reste d'hydrocarbure aromatique en $C_6$-$C_{15}$ éventuellement substitué un reste d'hydrocarbure arylaliphatique en $C_7$-$C_{14}$ éventuellement substitué ou un reste hétérocyclique à 5 ou 6 chaînons éventuellement substitué qui peut encore être accolé avec un noyau benzénique,

$R_2$ représente un reste alkyle en $C_1$-$C_{20}$ éventuellement substitué un reste cycloalkyle en $C_3$-$C_{16}$ éventuellement substitué et un reste arylalkyle en $C_7$-$C_{14}$ éventuellement substitué et

n est un nombre entier de 1 à 3

dans laquelle dans le cas où n = 2 ou 3 il y a au moins 2 atomes de carbone entre deux groupes uréthannes reliés au reste $R_1$ par réaction

a) d'urée ou de polyurets de formule

$H_2N$-$(CO$-$NH)_m$-$H$

dans laquelle

m représente un nombre entier de 1 à 4 ou bien de mélanges de ces composés avec

b) des amines primaires de formule

$R_1(NH_2)_n$

dans laquelle

$R_1$ et n ont la signification déjà indiquée et

c) des alcools de formule

$R_2OH$

dans laquelle $R_2$ a la signification délà indiquée dans un domaine de températures de 120 à 350°C caractérisé en ce que l'on utilise simultanément comme autres partenaires de réaction

d) des uréthannes non N-substitués de formule

$R_3$-$O$-$CO$-$NH_2$

dans laquelle $R_3$ correspond à la définition de $R_2$ les restes $R_3$ et $R_2$ étant identiques ou différents ou bien dans laquelle $R_3$ représente un reste d'hydrocarbure aromatique éventuellement substitué par le chlore ou par un alkyle en $C_1$-$C_4$ ayant au total 6 à 15 atomes de carbone et/ou

e) des urées ou des polyurées linéaires N-mono- ou N N'-disubstituées d'un poids moléculaire maximal de

2000 présentant éventuellement des groupes uréthannes ou des groupes amino primaires terminaux dont les groupes urées, les groupes uréthamnes ou les groupes amino sont reliés ensemble par des restes d'hydrocarbures ou bien dont les groupes urées sont substitués par des restes d'hydrocarbures qui correspondent au reste $R_1$ et dont les groupes uréthannes terminaux éventuellement présents sont substitués sur l'atome d'oxygène par des restes d'hydrocarbures qui correspondent au reste $R_2$

à l'exclusion de la préparation d'aryl-mono-, -di- et/ou -polyuréthannes par réaction de carbamates de O-alkyle, de O-cycloalkyle ou de O-arylalkyle éventuellement substitués avec des mono-, di- et/ou polyamines aromatiques primaires en présence d'alcools et d'urée sans l'utilisation simultanée des produits de départ mentionnés sous e), les partenaires de réaction a) à e) étant mélangés ensemble pour la mise en oeuvre du procédé et chauffés à la température de réaction de 120 à 350°C; et le composant amine b), y compris l'amine correspondant au composant amine b) liée chimiquement dans posant e), étant utilisé à raison de 0,5 à 4 fois la quantité stoechiométrique; et le composant alcool c), y compris l'alcool lié chimiquement dans les composants d) et e), dans le cas où celui-ci correspond au composant alcool c), étant utilisé à raison de 1 à 10 fois la quantité stoechiométrique, chaque fois par rapport aux groupes carbonyles présents dans les composants a), d) et e); et les composants d) et e) étant utilisés en quantité de 0 à 300 % en poids, par rapport à la quantité du composant a); avec la restriction que la quantité totale des composants d) et e) est d'au moins 10 % en poids.

2. Procédé selon la revendication 1 caractérisé en ce que l'on effectue la réaction en présence de catalyseurs d'estérification des acides carboxyliques comme catalyseurs.

3. Procédé selon les revendications 1 et 2 caractérisé en ce que l'on effectue la réaction en présence de solvants polaires.

4. Procédé selon la revendication 3 caractérisé en ce que l'on utilise comme solvant l'ε -caprolactame.